(19) Europäisches Patentamt
European Patent Office
Office européen des brevets

(11) Veröffentlichungsnummer: **0 322 362 B1**

(12) # EUROPÄISCHE PATENTSCHRIFT

(45) Veröffentlichungstag der Patentschrift: **08.05.91 Patentblatt 91/19**

(51) Int. Cl.$^5$: **C10M 135/36,** C07D 285/12

(21) Anmeldenummer: **88810861.0**

(22) Anmeldetag: **14.12.88**

(54) **Thiadiazolderivate als Schmierstoffadditive.**

Die Akte enthält technische Angaben, die nach dem Eingang der Anmeldung eingereicht wurden und die nicht in dieser Patentschrift enthalten sind.

(30) Priorität: **23.12.87 CH 5033/87**

(43) Veröffentlichungstag der Anmeldung: **28.06.89 Patentblatt 89/26**

(45) Bekanntmachung des Hinweises auf die Patenterteilung: **08.05.91 Patentblatt 91/19**

(84) Benannte Vertragsstaaten: **BE DE FR GB IT**

(56) Entgegenhaltungen:
**US-A- 2 765 289**
**US-A- 2 799 651**
**US-A- 4 704 426**
**CROATICA CHEMICA ACTA, Band 37, Nr. 4, 1965, Seiten 215-221, Zagreb, JU; J. KOBE et al.: "The Mannich reaction for 2,5-Dimercapto-1,3,4-thiadiazole"**

(73) Patentinhaber: **CIBA-GEIGY AG**
**Klybeckstrasse 141**
**CH-4002 Basel (CH)**

(72) Erfinder: **Camenzind, Hugo, Dr.**
**Avenue Général Guisan 42**
**CH-1700 Fribourg (CH)**

EP 0 322 362 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft 2,5-Dimercapto-1,3,4-thiadiazole enthaltende Zusammensetzungen und ihre Verwendung als Schmierstoffadditive oder als Additive in Hydraulikflüssigkeiten, sowie neue 2,5-Dimercapto-1,3,4-thiadiazole.

Auf dem Gebiet der Schmiermitteladditive wird zunehmend auf phosphor- und aschefreie Zusätze abgezielt.

Aus der US 2 765 289 sind 2,5-Dimercapto-1,3,4-thiadiazole bekannt, die an der 2- und 5-Stellung mit einer Alkyldiarylaminomethylgruppe substituiert sind. Diese Verbindungen werden als Korrosions-Inhibitoren in Schmierölen beschrieben.

Diese Produkte konnten aber nicht vollumfänglich befriedigen. So war der Schutz vor Reibabnutzung und vor allem die Neutralisierung von aktivem, für Kupfer und Silber korrosivem Schwefel unbefriedigend.

Aus J. Kobe, A. Pollak, A. Stanovnik und M. Tisler, Croatica Chemica Acta 37 (1965), Seiten 215-221, ist eine Mannich-Reaktion an 2,5-Dimercapto-1,3,4-thiadiazol bekannt. Es wird 2,5-Dimercapto-1,3,4-thiadiazol beschrieben, das in 2- und 4-Stellung, beispielsweise mit einem N-Morpholinomethylrest, substituiert ist.

Es wurde nun gefunden, dass bestimmte 2,5-Dimercapto-1,3,4-thiadiazole, die sowohl phosphor- als auch aschefrei sind, neben ihrer guten Eignung als Antioxidantien, Verschleissschutz- und Hochdruckadditive auch Metallteile, und hierbei insbesondere Kupfer, wirksam vor Korrosion und dem korrosiven Einfluss von Schwefel zu schützen vermögen, d.h. eine besonders breite, polyvalente Wirkung als Schmierstoff- oder Hydraulikflüssigkeitzusatz zeigen.

Die vorliegende Erfindung betrifft demnach Schmierstoffzusammensetzungen oder Hydraulikflüssigkeitzusammensetzungen enthaltend wenigstens einen Schmierstoff oder eine Hydraulikflüssigkeit und mindestens ein 2,5-Dimercapto-1,3,4-thiadiazol der allgemeinen Formel

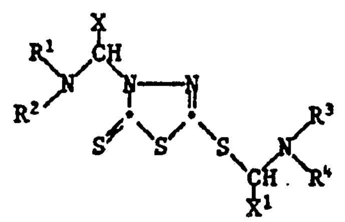

(I)

wobei $R^1$, $R^2$, $R^3$ und $R^4$ gleich oder verschieden sind und die Bedeutung von Alkyl mit 1 bis 24 C-Atomen, Alkenyl mit 3 bis 12 C-Atomen, unsubstituiertem oder im Phenylrest $C_1$-$C_4$-alkylsubstituiertem Phenyl-($C_1$-$C_4$)-alkyl haben oder $R^1$ und $R^2$ und/oder $R^3$ und $R^4$ stellen mit dem sie verbindenden N-Atom einen unsubstituierten oder $C_1$-$C_4$-Alkyl substituierten Morpholin-, Piperidin-, Pyrrolidin-, Piperazin-, Methylpiperazin- oder Perhydroazepinrest dar, und X und $X^1$ gleich oder verschieden sind und H, Alkyl mit 1 bis 23 C-Atomen, Phenyl, Phenyl-($C_1$-$C_4$)-alkyl oder mit $C_1$-$C_{12}$-Alkyl, $C_1$-$C_{12}$-Alkoxy, Hydroxy, Di-($C_1$-$C_4$-alkyl)-amino, Halogen und/oder Nitro substituiertes Phenyl bedeuten.

Mit Alkyl mit 1 bis 24 C-Atomen werden geradkettige oder verzweigte Alkylgruppen umfasst, die beispielsweise Methyl, Ethyl, Propyl, Isopropyl, n-Butyl, Isobutyl, 2-Butyl, t-Butyl, Pentyl, Isopentyl, Hexyl, Heptyl, 3-Heptyl, n-Octyl, 2-Ethylhexyl, Nonyl, Decyl, Undecyl, Dodecyl, Tridecyl, Tetradecyl, Pentadecyl, Hexadecyl, Heptadecyl, Octadecyl oder Eicosyl einschliessen.

Sinngemäss können Alkylgruppen mit 1 bis 4 oder 1 bis 12 C-Atomen oben genannter Aufzählung entnommen werden.

Bei Alkenyl mit 3 bis 12 C-Atomen sind Beispiele für entsprechende Substituenten Allyl, 2-Methallyl, 2-Butenyl, 2-Hexenyl, Decenyl oder 10-Undecyl. Allyl wird bevorzugt.

Gegebenenfalls substituiertes Phenyl($C_1$-$C_4$)alkyl kann beispielsweise Benzyl, α-Methylbenzyl, α,α-Dimethylbenzyl oder t-Butylbenzyl sein.

Haben X und $X^1$ die Bedeutung von Alkyl mit 1 bis 23 C-Atomen, gelten sinngemäss die oben genannten Beispiele, wobei noch die Methylgruppe bevorzugt erwähnt werden soll.

Unter $C_1$-$C_{12}$-Alkoxy können in beispielhafter Aufzählung Ethoxy, Butoxy, t-Butoxy, Octoxy, 2-Ethyl-hexyloxy usw. genannt werden.

Mit Halogen ist vor allem Fluor, Chlor oder Brom und insbesondere Chlor gemeint.

Eine zweckmässige Zusammensetzung enthält wenigstens eine Verbindung der Formel I, worin $R^1$, $R^2$, $R^3$ und $R^4$ $C_2$-$C_{18}$-Alkyl oder Phenyl($C_1$-$C_4$)alkyl, oder $R^1$ und $R^2$ und/oder $R^3$ und $R^4$ zusammen mit dem sie verbindenden N-Atom einen unsubstituierten oder $C_1$-$C_4$-Alkyl substituierten Morpholin-, Piperidin-, Piperazin- oder Perhydroazepinrest, X und $X^1$ H oder Alkyl mit 1 bis 10 C-Atomen, Phenyl oder mit –OH substituiertes

Phenyl und insbesondere H bedeutet.

Bevorzugte Zusammensetzungen enthalten eine Verbindung der Formel I, worin $R^1$, $R^2$, $R^3$ und/oder $R^4$ eine Alkylgruppe mit 2 bis 18 C-Atomen oder eine Phenylmethylgruppe darstellen können.

Bevorzugt sind beispielsweise Zusammensetzungen in denen $R^1$ und $R^3$ gleich und $R^2$ und $R^4$ gleich sind.

Mit $R^1$ und $R^3$ als Alkyl mit 2-8 C-Atomen und $R^2$ und $R^4$ als Phenylmethyl werden andere bevorzugte Verbindungen, die in der Zusammensetzung enthalten sein können, beschrieben.
Weiters können $R^1$, $R^2$, $R^3$ und $R^4$ gleich sein und vorzugsweise jeweils 2-Ethylhexyl oder Allyl darstellen.
In zweckmässiger Form enthalten die Zusammensetzungen Verbindungen der Formel I, in denen X und $X^1$ gleich sind und insbesondere H, Alkyl mit 1 bis 10 C-Atomen, Phenyl oder mit –OH substituiertes Phenyl darstellen. Bevorzugt soll X und $X^1$ H darstellen.

Die Verbindungen der Formel I lassen sich auf an sich bekannte Weise herstellen.
Beispielsweise wird zuerst das 2,5-Dimercapto-1,3,4-thiazol durch Umsetzung von einem Mol Hydrazin oder Hydrazinsalz mit zwei Mol Schwefelkohlenstoff in alkalischem Milieu und anschliessendem Ansäuern hergestellt (US 4 599 425). Aus dem 2,5-Dimercapto-1,3,4-thiazol sind die Verbindungen der Formel I zum Beispiel nach der erwähnten Literaturstelle Kobe et al. Croat. Chem. Acta 37 (1965) 215-221 herstellbar.

Zur Durchführung des Verfahrens werden die Ausgangsstoffe, demnach das 2,5-Dimercapto-1,3,4-thiazol, das entsprechende Amin und das entsprechende Aldehyd zusammengegeben und bei Temperaturen, die an sich unkritisch sind und normalerweise zwischen Raumtemperatur und Rückflusstemperatur liegen und zweckmässig bei Temperaturen von 50° bis 70°C, während Zeiträumen bis zu 24 h umgesetzt.
In der Regel wird ein Lösungsmittel und insbesondere ein nicht polares Lösungsmittel angewendet. Beispielsweise können Tetrachlormethan, aromatische und gesättigte aliphatische Kohlenwasserstoffe, wie z.B. Benzine, Cyclohexan, Methylcyclohexan, Dekalin, Terpene, Benzol, Toluol oder Xylole, sowie geeignete Mischungen davon als Lösungsmittel genannt werden.

Um optimale Umsätze und Ausbeuten zu erreichen, sollen das 2,5-Dimercapto-1,3,4-thiadiazol, das Aldehyd und das Amin ein Mengen-Verhältnis von 1 : 3 : 3 bis 1 : 2 : 2 angewendet werden, wobei das Verhältnis 1 : 2 : 2 vorzuziehen ist.

Entsprechend der gewünschten Endverbindung können die Aldehyde aus der Reihe der aliphatischen oder aromatischen mit 1 bis 24 C-Atomen sein und können mit Alkoxy-, Hydroxy-, Mercapto- und/oder Nitrogruppen substituiert sein. Namentlich genannt werden können beispielsweise das bevorzugte Formaldehyd, weiters Acetaldehyd, Benzaldehyd, 2-Ethyl-hexylaldehyd, Butyraldehyd, Oenanthaldehyd, Caprinaldehyd, Salicylaldehyd und darüberhinaus gegebenenfalls auch Laurylaldehyd, Acetylsalicylaldehyd, ortho-Chlorbenzaldehyd, para-Chlorbenzaldehyd, Zimtaldehyd, para-Hydroxybenzaldehyd, Octylaldehyd, Decylaldehyd, p-Methoxybenzaldehyd, Phenylacetaldehyd oder 2,5-Dimethoxybenzaldehyd.

Die weiters zur Anwendung gelangenden Amine können Dialkyl-, Dialkenyl-, Monoalkylmonoarylalkyl- oder Diarylalkylamine sein. Beispiele sind Di-n-butylamin, Di-iso-butylamin, Dihexylamin, Dibenzylamin, Benzylmethylamin, Bis-2-ethyl-hexylamin, Dioctylamin, Di-iso-octylamin, Diallylamin usw., wobei Bis-2-ethyl-hexylamin und Diallylamin als bevorzugt gelten können.

Besonders bevorzugte Verbindungen, die auch nach den beschriebenen Verfahren erhältlich sind, neue Verbindungen darstellen und sich besonders zur Verwendung in Schmierstoffzusammensetzungen eignen, sind in beispielhafter Aufzählung :
N,S-Bis(dipropylaminomethyl)-2,5-dimercapto-1,3,4-thiadiazol,
N,S-Bis(dibutylaminomethyl)-2,5-dimercapto-1,3,4-thiadiazol,
N,S-Bis(dihexylaminomethyl)-2,5-dimercapto-1,3,4-thiadiazol,
N,S-Bis(benzylethylaminomethyl)-2,5-dimercapto-1,3,4-thiadiazol,
N,S-Bis[bis(2-ethyl-hexyl)-aminomethyl]-2,5-dimercapto-1,3,4-thiadiazol,
N,S-Bis(dibenzylaminomethyl)-2,5-dimercapto-1,3,4-thiadiazol,
N,S-Bis(diallylaminomethyl)-2,5-dimercapto-1,3,4-thiadiazol,
N,S-Bis(ditridecylaminomethyl)-2,5-dimercapto-1,3,4-thiadiazol,
N,S-Bis(dioctadecylaminomethyl)-2,5-dimercapto-1,3,4-thiadiazol,

Die erfindungsgemäss umfassten Verbindungen können aufgrund der guten Oellöslichkeit in Schmierstoffen generell und in Mineralölen und synthetischen oder halbsynthetischen Oelen besonders gut eingesetzt werden. Dabei entfalten sie die Wirkung als Reibungsschutz oder Extremdruck/Verschleissschutz-Additive (EP/AW), Antioxidans (AO) und Korrosionsinhibitor (CI). Geeignet sind die Verbindungen auch als Zusatz zu Hydraulikflüssigkeiten. Ueblicherweise enthalten die Zusammensetzungen 0,01 bis 10 Gew.-%, zweckmässig 0,05 bis 5 Gew.-% und bevorzugt 0,1 bis 3 Gew.-%, jeweils bezogen auf den Schmierstoff oder die Hydraulikflüssigkeit mindestens einer Verbindung der Formel I nach vorliegender Erfindung.

Die in Frage kommenden Schmierstoffe und Hydraulikflüssigkeiten sind dem Fachmann geläufig und z.B. im "Schmiermittel Taschenbuch" (Hüthig Verlag, Heidelberg, 1974) resp. in "Ullmanns Encyclopädie der technischen Chemie" Bd. 13, Seiten 85-94 (Verlag Chemie, Weinheim, 1977) oder von D. Klamann in "Schmierstoffe und verwandte Produkte" Verlag Chemie, Weinheim (1982) beschrieben.

Der Schmierstoff kann beispielsweise ein Oel, basierend auf einem Mineralöl oder einem synthetischen Oel, oder gegebenenfalls ein Fett sein. Der Ausdruck Mineralöl umfasst alle Mineralöle für Schmierzwecke, wie Mineralöle auf Kohlenwasserstoffbasis. Synthetische Oele können beispielsweise aliphatische oder aromatische Carboxylester, polymere Ester, Polyalkylenoxide, Phosphorsäureester, Poly-α-olefine, Silicone, Glykole, Polyglykole oder Polyalkylenglykole sein.

Die Schmierstoffe oder Hydraulikflüssigkeiten können zusätzlich andere Additive enthalten, die zugegeben werden, um die Grundeigenschaften von Schmierstoffen oder allgemein der "functional fluids" noch weiter zu verbessern ; dazu gehören Antioxidantien, Metallpassivatoren, Rostinhibitoren, Viskositätsindex-Verbesserer, Stockpunkterniedriger, Dispergiermittel, Detergentien, weitere Hochdruck-Zusätze sowie andere Verschleissschutz-Additive.

1. Alkylierte Monophenole

2,6-Di-tert-butylphenol, 2,6-Di-tert-butyl-4-methylphenol, 2,6-Di-tert-butylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, o-tert-Butylphenol.

2. Alkylierte Hydrochinone

2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol.

3. Hydroxylierte Thiodiphenylether

2,2′-Thio-bis-(6-tert-butyl-4-methylphenol), 2,2′-Thio-bis-(4-octylphenol), 4,4′-Thio-bis-(6-tert-butyl-3-methylphenol), 4,4′-Thio-bis-(6-tert-butyl-2-methylphenol).

4. Alkyliden-Bisphenole

2,2′-Methylen-bis-(6-tert-butyl-4-methylphenol), 2,2′-Methylen-bis-(6-tert-butyl-4-ethylphenol), 2,2′-Methylen-bis-[4-methyl-6-(α-methylcyclohexyl)-phenol], 2,2′-Methylen-bis-(4-methyl-6-cyclohexylphenol), 2,2′-Methylen-bis-(6-nonyl-4-methylphenol), 2,2′-Methylen-bis-(4,6-di-tert-butylphenol), 2,2′-Ethyliden-bis-(4,6-di-tert-butylphenol), 2,2′-Ethyliden-bis-(6-tert-butyl-4- oder -5-iso-butylphenol), 2,2′-Methylen-bis-[6-(α-methylbenzyl)-4-nonylphenol], 2,2′-Methylen-bis-[6-(α,α-dimethylbenzyl)-4-nonylphenol], 4,4′-Methylen-bis-(2,6-di-tert-butylphenol), 4,4′-Methylen-bis-(6-tert-butyl-2-methylphenol), 1,1-Bis-(5-tert-butyl-4-hydroxy-2-methylphenyl)-butan, 2,6-Di-(3-tert-butyl-5-methyl-2-hydroxybenzyl)-4-methylphenol, 1,1,3-Tris-(5-tert-butyl-4-hydroxy-2-methylphenyl)-3-n-dodecylmercaptobutan, Ethylenglycol-bis-[3,3-bis-(3′-tert-butyl-4′-hydroxyphenyl)-butyrat], Bis-(3-tert-butyl-4-hydroxy-5-methylphenyl)-dicyclopentadien, Bis-[2-(3′-tert-butyl-2′-hydroxy-5′-methyl-benzyl)-6-tert-butyl-4-methyl-phenyl]-terephthalat.

5. Benzylverbindungen 1,3,5-Tri-(3,5-di-tert-butyl-4-hydroxybenzyl)-2,4,6-trimethylbenzol,
Bis-(3,5-di-tert-butyl-4-hydroxybenzyl)-sulfid,
3,5-Di-tert-butyl-4-hydroxybenzyl-mercaptoessigsäure-isooctylester,
Bis-(4-tert-butyl-3-hydroxy-2,6-dimethylbenzyl)-dithiol-terephthalat,
1,3,5-Tris-(3,5-di-tert-butyl-4-hydroxybenzyl)-isocyanurat,
1,3,5-Tris-(4-tert-butyl-3-hydroxy-2,6-dimethylbenzyl)-isocyanurat,
3,5-Di-tert-butyl-4-hydroxybenzyl-phosphonsäure-dioctadecylester,
3,5-Di-tert-butyl-4-hydroxybenzyl-phosphonsäure-monoethylester, Calcium-salz.

6. Acylaminophenole 4-Hydroxy-laurinsäureanilid, 4-Hydroxy-stearinsäureanilid,
2,4-Bis-octylmercapto-6-(3,5-di-tert-butyl-4-hydroxyanilino)-s-triazin,
N-(3,5-di-tert-butyl-4-hydroxyphenyl)-carbaminsäureoctylester.

7. Ester der β-(3,5-Di-tert-butyl-4-hydroxyphenyl)-propionsäure mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Bis-hydroxyethyl-oxalsäurediamid.

8. Ester der β-(5-tert-butyl-4-hydroxy-3-methylphenyl)-propionsäure mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Di-hydroxyethyl-oxalsäurediamid.

9. Amide der β -(3,5-Di-tert-butyl-4-hydroxphenyl)-propionsäure, wie z.B.
N,N'-Bis-(3,5-di-tert-butyl-4-hydroxyphenylpropionyl)-hexamethylendiamin,
N,N'-Bis-(3,5-di-tert-butyl-4-hydroxyphenylpropionyl)-trimethylendiamin,
N,N'-Bis-(3,5-di-tert-butyl-4-hydroxyphenylpropionyl)-hydrazin.

Beispiele für aminische Antioxidantien : N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl-/tert-Octyldiphenylaminen, 2,3-Dihydro-3,3-dimethyl-4H-1,4-benzothiazin, Phenothiazin, N-Allylphenothiazin.

Beispiele für weitere Antioxidantien : Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind : Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5'-Methylenbisbenztriazol, 4,5,6,7-Tetrahydrobenztriazol, Salicyliden-propylendiamin, Salicylaminoguanidin und dessen Salze.

Beispiele für Rost-Inhibitoren sind :

a) Organische Säuren, ihre Ester, Metallsalze und Anhydride, z.B. :
N-Oleoyl-sarcosin, Sorbitan-mono-oleat, Blei-naphthenat, Alkenylbernsteinsäureanhydride, z.B. Dodecenylbernsteinsäure-anhydrid, Alkenylbernsteinsäure-Teilester und -Teilamide, 4-Nonylphenoxy-essigsäure.

b) Stickstoffhaltige Verbindungen, z.B. :

I. Primäre, sekundäre oder tertiäre aliphatische oder cycloaliphatische Amine und Amin-Salze von organischen und anorganischen Säuren, z.B. öllösliche Alkylammoniumcarboxylate.

II. Heterocyclische Verbindungen, z.B. :
substituierte Imidazoline und Oxazoline.

c) Phosphorhaltige Verbindungen, z.B. :

Aminsalze von Phosphorsäurepartialestern oder Phosphonsäurepartialestern, Zinkdialkyldithiophosphate.
d) Schwefelhaltige Verbindungen, z.B.:
Barium-dinonylnaphthalin-sulfonate, Calciumpetroleum-sulfonate.

Beispiele für Viskositätsindex-Verbesserer sind: Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind: Polymethacrylate, alkylierte Naphthalinderivate.

Beispiele für Dispergiermittel/Tenside sind: Polybutenylbernsteinsäureamide oder -imide, Polybutenylphosphonsäurederivate, basische Magnesium-, Calcium-, und Bariumsulfonate und -phenolate.

Beispiele für Verschleissschutz-Additive sind: Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Schliesslich kann die Schmierstoffzusammensetzung auch ein Co-Schmiersystem enthaltend übliche Mengen von Festschmierstoffen, wie Graphit, Molybdändisulfid, Bornitrid oder Tetrafluorethylen (Teflon) enthalten.

Die vorliegende Erfindung umfasst auch neue 2,5-Dimercapto-1,3,4-thiadiazole der allgemeinen Formel I

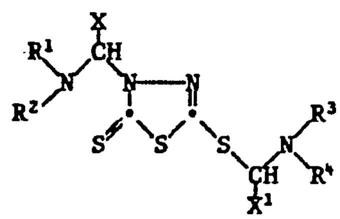

wobei $R^1$, $R^2$, $R^3$ und $R^4$ gleich oder verschieden sind und die Bedeutung von Alkyl mit 1 bis 24 C-Atomen, Alkenyl mit 3 bis 12 C-Atomen, unsubstituiertem oder im Phenylrest $C_1$-$C_4$-alkylsubstituiertem Phenyl-($C_1$-$C_4$)-alkyl haben oder $R^1$ und $R^2$ und/oder $R^3$ und $R^4$ mit dem sie verbindenden N-Atom einen unsubstituierten oder durch $C_1$-$C_4$-Alkyl substituierten Morpholin-, Piperidin-, Pyrrolidin-, Piperazin-, Methylpiperazin- oder Perhydroazepinrest darstellen, und X und $X^1$ gleich oder verschieden sind und H, Alkyl mit 1 bis 23 C-Atomen, Phenyl, Phenyl-($C_1$-$C_4$)-alkyl oder mit $C_1$-$C_{12}$-Alkyl, $C_1$-$C_{12}$-Alkoxy, Hydroxy, Di-($C_1$-$C_4$)-alkylamino, Halogen und/oder Nitro substituiertes Phenyl bedeuten, mit der Massgabe, dass nicht $R^1$ mit $R^2$ und $R^3$ mit $R^4$ gleichzeitig mit dem sie jeweils verbindenden N-Atom einen Morpholinorest darstellen, wenn X = H ist.

Bevorzugte Verbindungen der Formel I sind solche worin $R^1$, $R^2$, $R^3$ und/oder $R^4$ eine Alkylgruppe mit 2 bis 18 C-Atomen oder eine Phenylmethylgruppe darstellen, weiters Verbindungen der Formel I worin $R^1$ und $R^3$ gleich sind und $R^2$ und $R^4$ gleich sind und die angegebene Bedeutung haben und ferner Verbindungen der Formel I worin $R^1$ und $R^3$ Alkyl mit 2-8 C-Atomen darstellt und $R^2$ und $R^4$ Phenylmethyl darstellt.

Schliesslich sind Verbindungen der Formel I besonders bevorzugt, worin $R^1$, $R^2$, $R^3$ und $R^4$ gleich sind und insbesondere jeweils 2-Ethyl-hexylamin darstellen.

Weitere Verbindungen der Formel I, die zu den zweckmässigen Ausführungsformen gehören, sind solche, worin X und $X^1$ gleich sind und H, Alkyl mit 1 bis 10 C-Atomen, Phenyl oder mit –OH substituiertes Phenyl darstellen und bevorzugt sind Verbindungen der Formel I, worin X und $X^1$ H darstellen.

Sinngemäss sind solche Verbindungen zweckmässig oder bevorzugt, welche die allgemeine Formel I aufweisen und zu den zweckmässigen oder bevorzugten Zusammensetzungen führen.

Vorliegende Erfindung betrifft auch die Verwendung von Verbindungen der Formel I, wie oben genannt, als Schmierstoff-Additive oder Additive für Hydraulikflüssigkeiten.

Vorliegende Erfindung wird anhand der nachfolgenden Beispiele noch näher erläutert. Prozent und Teile beziehen sich darin und in der übrigen Beschreibung auf das Gewicht, sofern nichts anderes angegeben ist.

Beispiel 1: N,S-Bis(di-n-hexylaminomethyl)-2,5-dimercapto-1,3,4-thiadiazol

Zu einer Lösung von 18,9 g (0,1 mol) Di-n-hexylamin in 50 ml Toluol wird eine Suspension von 7,7 g (0,051 mol) 2,5-Dimercapto-1,3,4-thiadiazol und 9,6 g wässerigem Formaldehyd (36%, 0,12 mol) gegeben. Das Reaktionsgemisch wird ca. 15 Std. bei 50°C gerührt. Dann wird das Wasser von der organischen Phase getrennt. Diese wird mit etwas Wasser neutral gewaschen und eingedampft: Man erhält 26 g orange-rötliches Oel (95% d. Th.), das der Titelverbindung entspricht.

In analoger Weise wurden die Verbindungen der Beispiele 2-9 hergestellt. Die Verbindungen der Beispiele

1-9 weisen die allgemeine Formel

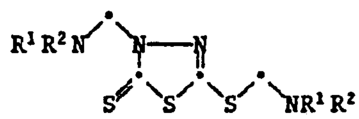

auf und die Bedeutung von $NR^1R^2$ lässt sich der nachfolgenden Tabelle entnehmen.

| Bsp. | $NR^1R^2$ | Ausbeute (% d. Th.) | Aspekt | $R^1R^2N$-$CH_2$-N/S $^1H$-NMR [$CDCl_3$, ppm] | Analyse [berechnet / gefunden] C | H | N | S |
|---|---|---|---|---|---|---|---|---|
| 1 | Di-n-hexylamin 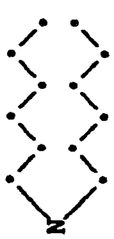 | 95 | orange-rötliches Oel | 5,15(2H,s) | 61,71<br>62,10 | 10,36<br>10,18 | 10,28<br>10,63 | 17,65<br>16,95 |
| 2 | Di-n-propylamin 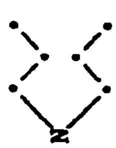 | 58 | braunes Oel | 5,41(2H,s) | 51,02<br>50,79 | 8,56<br>8,29 | 14,88<br>14,99 | 25,54<br>24,59 |
| 3 | Di-n-butylamin 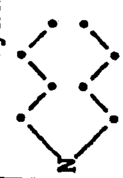 | 88 | braunes Oel | 5,14(2H,s) | 55,51<br>55,54 | 9,32<br>9,27 | 12,95<br>13,21 | 22,23<br>21,54 |
| 4 | Ethyl-benzylamin 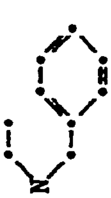 | 93 | braunes Oel | 5,11(2H,s, breit) | - | - | - | - |

| Bsp. | $NR^1R^2$ | Ausbeute (% d. Th.) | Aspekt | $R^1R^2N-CH_2-N/S$ $^1H$-NMR [$CDCl_3$, ppm] | Analyse [berechnet / gefunden] C | H | N | S |
|---|---|---|---|---|---|---|---|---|
| 5 | Dibenzylamin 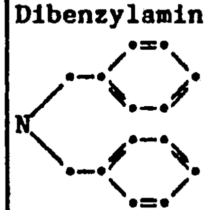 | 93 | gelb-oranges hoch-viskoses Oel | 5,01(2H,2xs) | 67,57<br>66,93 | 5,67<br>5,57 | 9,85<br>10,41 | 16,91<br>17,19 |
| 6 | Di-2-ethylhexyl-amin 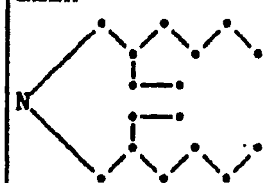 | 96 | gelbes Oel | 5,13(2H,s) | 65,80<br>65,79 | 11,04<br>11,17 | 8,53<br>8,77 | 14,64<br>14,18 |
| 7 | Diallyl-amin 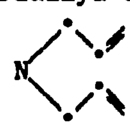 | 90 | braunes Oel | 5,22 | 52,14<br>52,16 | 6,56<br>6,79 | 15,20<br>15,83 | 26,1<br>25,5 |

| Bsp. | $NR^1R^2$ | Ausbeute (% d. Th.) | Aspekt | $R^1R^2N-CH_2-N/S$ $^1H$-NMR [$CDCl_3$, ppm] | Analyse [berechnet / gefunden] C | H | N | S |
|---|---|---|---|---|---|---|---|---|
| 8 | Di-tridecyl-amin $N(C_{13}H_{27})_2$ | 99 | oranges Oel | 5,14 | 71,73<br>73,44 | 12,04<br>12,45 | 5,98<br>5,79 | 10,26<br>8,5 |
| 9 | Di-octadecyl-amin $N(C_{18}H_{37})_2$ | 99 | gelbes Pulver Smp. 40-49°C | 5,14 | 74,93<br>74,78 | 12,58<br>12,46 | 4,6<br>4,76 | 7,89<br>7,73 |

Beispiel 10 : Test auf Verschleissschutz

Zur Prüfung auf Eignung als Verschleissschutzadditiv wird die ASTM-Standardmethode D-2783-81 unter Verwendung des Shell-Vierkugelapparates herangezogen. Als Basisöl wird Catenex®P941 der Fa. Shell verwendet. Ermittelt wird der mittlere Verschleiss-Narben-Durchmesser (Wear Scar Diameter) bei einer Last von 40 kg während 1 Std. (in mm).

| Verbindung aus Beispiel | Additivmenge (Gew.-%) bezogen auf Basisöl | WSD (mm) |
|---|---|---|
| ohne Additiv | - | 0,9 |
| 6 | 1 | 0,55 |
| 7 | 1 | 0,64 |
| 8 | 1 | 0,53 |
| 9 | 1 | 0,50 |

Beispiel 11 : Test auf Stabilisierung gegen oxidativen Abbau

(TFOUT : Thin Film Oxygen Uptake Test)

Dieser Test ist eine modifizierte Form des Rotary Bomb Test für Mineralöle (ASTM D 2272). Eine detaillierte Beschreibung findet sich bei C.S.Ku, S.M.Hsu, Lubrication Engineering 40 (1984) 75-83. Das Testöl ist in diesem Fall ein kommerzielles Motorenöl 15W40, mit ca. der Hälfte des üblichen Gehaltes an Zinkdithiophosphaten (0,75% ZnDTP, 550 ppm P, 1160 ppm Zn). Das zu testende Additiv wird im Oel in Gegenwart von Wasser (2%), einer oxidierten/nitrierten Benzinfraktion (4%) und eines Gemisches von flüssigen Metallnaphthenaten (4%) bei 6,1 bar Sauerstoffdruck und 160°C auf seine stabilisierende Wirkung getestet. Das Wasser und die beiden flüssigen Katalysatoren für den Test werden unter der Bezeichnung Standard Reference Material 1817 vom National Bureau of Standards (NBS) bezogen, mit Bescheinigung für die Analyse. Der Test ist abgeschlossen, wenn im Druck/Zeit-Diagramm ein deutlicher Knick die einsetzende Oxidation am Ende der Induktionsperiode [min] anzeigt.

Eine lange Induktionsperiode bedeutet eine gute stabilisierende Wirkung des Additives.

| Verbindung aus Beispiel | Additivmenge (Gew.-%) bezogen auf Basisöl | Induktionsperiode (min) |
|---|---|---|
| ohne Additiv | - | 83 |
| 6 | 0,5 | 154 |
| 7 | 0,5 | 209 |
| 8 | 0,5 | 136 |
| 9 | 0,5 | 138 |

Beispiel 12 : Test auf Korrosionsschutz für Kupfer gegen aktiven Schwefel

Ein blank poliertes Kupferblech von 60 x 10 x 1 mm wird in Turbinenöl getaucht, das 50 ppm gelösten Schwefel sowie 0,2% des zu testenden Additivs enthält. Die Proben werden 2 Std. auf 100°C erwärmt. In einer zweiten Stufe werden die gleichen Kupferbleche mit Petrolether gespült und erneut in ein Turbinenöl mit 50 ppm aktivem Schwefel gestellt und 24 Std. auf 100°C erwärmt. Die Farbe der Bleche wird nach ASTM D 130 beurteilt durch Vergleich mit einer Standard-Farbtabelle. Die Beurteilung geschieht in 4 Stufen :

1 – kein Beschlag
2 – mässiger Beschlag
3 – starker Beschlag
4 – Korrosion

A und B bedeuten eine Feinunterteilung innerhalb der Zahlengruppen 1 bis 4 und bedeuten die Schattenbildung auf den Proben. In der qualitativen Beurteilung steht die Wertung A vor B.

| Verbindung aus Beispiel | Additiv (Gew.-%) bezogen auf Basisöl | Farbe 100° 2 Std. | 100° 24 Std. |
|---|---|---|---|
| ohne Additiv | - | 3B | - |
| 6 | 0,2 | 1A | 1B |

**Ansprüche**

1. Schmierstoffzusammensetzung oder Hydraulikflüssigkeitszusammensetzung, enthaltend wenigstens einen Schmierstoff oder eine Hydraulikflüssigkeit und mindestens ein 2,5-Dimercapto-1,3,4-thiadiazol der allgemeinen Formel I

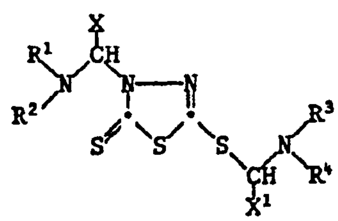

(I)

wobei $R^1$, $R^2$, $R^3$ und $R^4$ gleich oder verschieden sind und die Bedeutung von Alkyl mit 1 bis 24 C-Atomen, Alkenyl mit 3 bis 12 C-Atomen, unsubstituiertem oder im Phenylrest $C_1$-$C_4$-alkylsubstituiertem Phenyl-($C_1$-$C_4$)-alkyl haben oder $R^1$ und $R^2$ und/oder $R^3$ und $R^4$ stellen mit dem sie verbindenden N-Atom einen unsubstituierten oder $C_1$-$C_4$-Alkyl substituierten Morpholin-, Piperidin-, Pyrrolidin-, Piperazin-, Methylpiperazin-oder Perhydroazepinrest dar, und X und $X^1$ gleich oder verschieden sind und H, Alkyl mit 1 bis 23 C-Atomen, Phenyl, Phenyl-($C_1$-$C_4$)-alkyl oder mit $C_1$-$C_{12}$-Alkyl, $C_1$-$C_{12}$-Alkoxy, Hydroxy, Di-($C_1$-$C_4$-alkyl)-amino, Halogen und/oder Nitro substituiertes Phenyl bedeuten.

2. Zusammensetzung nach Anspruch 1 worin $R^1$, $R^2$, $R^3$ und $R^4$ $C_2$-$C_{18}$-Alkyl oder Phenyl($C_1$-$C_4$)alkyl, oder $R^1$ und $R^2$ und/oder $R^3$ und $R^4$ zusammen mit dem sie verbindenden N-Atom einen Morpholin-, Piperidin- oder Piperazinrest und X und $X^1$ H oder Alkyl mit 1 bis 10 C-Atomen, Phenyl oder mit –OH substituiertes Phenyl bedeutet.

3. Zusammensetzung nach Anspruch 1, worin $R^1$, $R^2$, $R^3$ und/oder $R^4$ eine Alkylgruppe mit 2 bis 18 C-Atomen, Allyl oder eine Phenylmethylgruppe darstellen.

4. Zusammensetzung nach Anspruch 1, worin $R^1$ und $R^3$ gleich sind und $R^2$ und $R^4$ gleich sind und die angegebene Bedeutung haben.

5. Zusammensetzung nach Anspruch 1, worin $R^1$ und $R^3$ Alkyl mit 2-8 C-Atomen darstellen und $R^2$ und $R^4$ Phenylmethyl darstellen.

6. Zusammensetzung nach Anspruch 1, worin $R^1$, $R^2$, $R^3$ und $R^4$ jeweils 2-Ethyl-hexyl darstellen.

7. Zusammensetzung nach Anspruch 1, worin X und $X^1$ gleich sind und H, Alkyl mit 1 bis 10 C-Atomen, Phenyl oder mit –OH substituiertes Phenyl darstellen.

8. Zusammensetzung nach Anspruch 1, worin X und $X^1$ H darstellen.

9. Zusammensetzung nach Anspruch 1, enthaltend N,S-Bis[bis(2-ethyl-hexyl)-amino-methyl]-2,5-dimercapto-1,3,4-thiadiazol und/oder N,S-Bis(diallylaminomethyl)-2,5-dimercapto-1,3,4-thiadiazol.

10. Zusammensetzung nach Anspruch 1, enthaltend 0,01 bis 10 Gew.-%, zweckmässig 0,05 bis 5 Gew.-% und vorzugsweise 0,1 bis 3 Gew.-%, bezogen auf den Schmierstoff, mindestens einer Verbindung der Formel I.

11. 2,5-Dimercapto-1,3,4-thiadiazole der allgemeinen Formel I

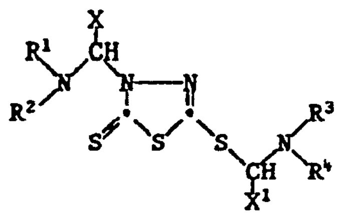

(I)

wobei $R^1$, $R^2$, $R^3$ und $R^4$ gleich oder verschieden sind und die Bedeutung von Alkyl mit 1 bis 24 C-Atomen, Alkenyl mit 3 bis 12 C-Atomen, unsubstituiertem oder im Phenylrest $C_1$-$C_4$-alkylsubstituiertem Phenyl-($C_1$-$C_4$)-alkyl haben oder $R^1$ und $R^2$ und/oder $R^3$ und $R^4$ stellen mit dem sie verbindenden N-Atom einen unsubstituierten oder $C_1$-$C_4$-Alkyl substituierten Morpholin-, Piperidin-, Pyrrolidin-, Piperazin-, Methylpiperazin- oder Perhydroazepinrest dar, und X und $X^1$ gleich oder verschieden sind und H, Alkyl mit 1 bis 23 C-Atomen, Phenyl, Phenyl-($C_1$-$C_4$)-alkyl oder mit $C_1$-$C_{12}$-Alkyl, $C_1$-$C_{12}$-Alkoxy, Hydroxy, Di-($C_1$-$C_4$-alkyl)-amino, Halogen und/oder Nitro substituiertes Phenyl bedeuten, mit der Massgabe, dass nicht $R^1$ mit $R^2$ und $R^3$ mit $R^4$ gleichzeitig mit dem sie verbindenden N-Atom einen Morpholinorest darstellen, wenn X = H ist.

12. Verbindungen der Formel I nach Anspruch 11, worin $R^1$, $R^2$, $R^3$ und $R^4$ $C_2$-$C_{18}$-Alkyl oder Phenyl($C_1$-$C_4$)alkyl, oder $R^1$ und $R^2$ und/oder $R^3$ und $R^4$ zusammen mit dem sie verbindenden N-Atom einen unsubstituierten oder $C_1$-$C_4$-Alkyl substituierten Morpholin-, Piperidin- oder Piperazinrest, X und $X^1$ H oder Alkyl mit 1 bis 10 C-Atomen, Phenyl oder mit –OH substituiertes Phenyl bedeutet.

13. Verbindungen der Formel I nach Anspruch 11, worin $R^1$, $R^2$, $R^3$ und/oder $R^4$ eine Alkylgruppe mit 2 bis 18 C-Atomen oder eine Phenylmethylgruppe darstellen.

14. Verbindungen der Formel I nach Anspruch 11, worin $R^1$ und $R^3$ gleich sind und $R^2$ und $R^4$ gleich sind und die angegebene Bedeutung haben.

15. Verbindungen der Formel I nach Anspruch 11, worin $R^1$ und $R^3$ Alkyl mit 2-8 C-Atomen darstellen und $R^2$ und $R^4$ Phenylmethyl darstellen.

16. Verbindungen der Formel I nach Anspruch 11, worin $R^1$, $R^2$, $R^3$ und $R^4$ jeweils 2-Ethyl-hexyl darstellen.

17. Verbindungen der Formel I nach Anspruch 11, worin X und $X^1$ gleich sind und H, Alkyl mit 1 bis 10 C-Atomen, Phenyl oder mit –OH substituiertes Phenyl darstellen.

18. Verbindungen der Formel I nach Anspruch 11, worin X und $X^1$ H darstellen.

19. Verbindungen der Formel I nach Anspruch 11 :
N,S-Bis[bis(2-ethyl-hexyl)-aminomethyl]-2,5-dimercapto-1,3,4-thiadiazol und
N,S-Bis(diallylaminomethyl)-2,5-dimercapto-1,3,4-thiadiazol.

20. Verwendung von Verbindungen der Formel I nach Anspruch 11 als Schmierstoff-Additive oder Additive für Hydraulikflüssigkeiten.

## Claims

1. A lubricant composition or hydraulic fluid composition containing at least one lubricant or one hydraulic fluid and at least one 2,5-dimercapto-1,3,4-thiadiazole of general formula I

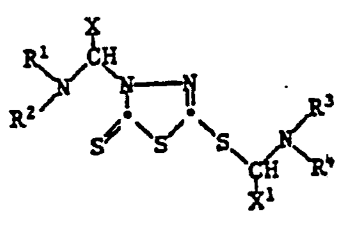

(I)

where $R^1$, $R^2$, $R^3$ and $R^4$ are identical or different and are alkyl having 1 to 24 C atoms, alkenyl having 3 to 12 C atoms or phenyl-($C_1$-$C_4$)alkyl which is unsubstituted or substituted in the phenyl radical by $C_1$-$C_4$alkyl, or $R^1$

and $R^2$ and/or $R^3$ and $R^4$, with the N atom to which they are bonded, are a morpholine, piperidine, pyrrolidine, piperazine, methylpiperazine or perhydroazepine radical which is unsubstituted or substituted by $C_1$-$C_4$alkyl, and X and $X^1$ are identical or different and are H, alkyl having 1 to 23 C atoms, phenyl, phenyl-($C_1$-$C_4$)alkyl or phenyl substituted by $C_1$-$C_{12}$alkyl, $C_1$-$C_{12}$alkoxy, hydroxyl, di($C_1$-$C_4$alkyl)amino, halogen and/or nitro.

2. A composition according to claim 1 in which $R^1$, $R^2$, $R^3$ and $R^4$ are $C_2$-$C_{18}$alkyl or phenyl-($C_1$-$C_4$)alkyl, or $R^1$ and $R^2$ and/or $R^3$ and $R^4$, together with the N atom to which they are bonded, are a morpholine, piperidine or piperazine radical, and X and $X^1$ are H, alkyl having 1 to 10 C atoms, phenyl or phenyl substituted by –OH.

3. A composition according to claim 1 in which $R^1$, $R^2$, $R^3$ and/or $R^4$ are an alkyl group having 2 to 18 C atoms, allyl or a phenylmethyl group.

4. A composition according to claim 1 in which $R^1$ and $R^3$ are identical and $R^2$ and $R^4$ are identical and as defined above.

5. A composition according to claim 1 in which $R^1$ and $R^3$ are alkyl having 2-8 C atoms and $R^2$ and $R^4$ are phenylmethyl.

6. A composition according to claim 1 in which $R^1$, $R^2$, $R^3$ and $R^4$ are each 2-ethylhexyl.

7. A composition according to claim 1 in which X and $X^1$ are identical and are H, alkyl having 1 to 10 C atoms, phenyl or phenyl substituted by –OH.

8. A composition according to claim 1 in which X and $X^1$ are H.

9. A composition according to claim 1 containing N,S-bis[bis(2-ethylhexyl)aminomethyl]-2,5-dimercapto-1,3,4-thiadiazole and/or N,S-bis(diallylaminomethyl)-2,5-dimercapto-1,3,4-thiadiazole.

10. A composition according to claim 1 containing 0.01 to 10% by weight, advantageously 0.05 to 5% by weight and preferably 0.1 to 3% by weight of at least one compound of formula I, based on the lubricant.

11. A 2,5-dimercapto-1,3,4-thiadiazole of general formula I

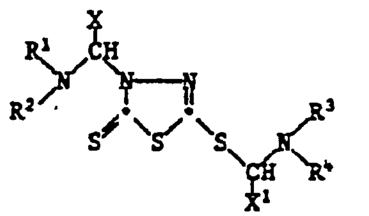

(I)

where $R^1$, $R^2$, $R^3$ and $R^4$ are identical or different and are alkyl having 1 to 24 C atoms, alkenyl having 3 to 12 C atoms or phenyl-($C_1$-$C_4$)alkyl which is unsubstituted or substituted in the phenyl radical by $C_1$-$C_4$alkyl, or $R^1$ and $R^2$ and/or $R^3$ and $R^4$, with the N atom to which they are bonded, are a morpholine, piperidine, pyrrolidine, piperazine, methylpiperazine or perhydroazepine radical which is unsubstituted or substituted by $C_1$-$C_4$alkyl, and X and $X^1$ are identical or different and are H, alkyl having 1 to 23 C atoms, phenyl, phenyl-($C_1$-$C_4$)alkyl or phenyl substituted by $C_1$-$C_{12}$alkyl, $C_1$-$C_{12}$alkoxy, hydroxyl, di($C_1$-$C_4$-alkyl)amino, halogen and/or nitro, with the proviso that $R^1$ and $R^2$, and $R^3$ and $R^4$, with the N atom to which they are bonded, are not simultaneously a morpholino radical when X = H.

12. A compound of formula I according to claim 11 in which $R^1$, $R^2$, $R^3$ and $R^4$ are $C_2$ -$C_{18}$alkyl or phenyl-($C_1$-$C_4$)alkyl, or $R^1$ and $R^2$ and/or $R^3$ and $R^4$, together with the N atom to which they are bonded, are a morpholine, piperidine or piperazine radical which is unsubstituted or substituted by $C_1$-$C_4$alkyl, and X and $X^1$ are H, alkyl having 1 to 10 C atoms, phenyl or phenyl substituted by –OH.

13. A compound of formula I according to claim 11 in which $R^1$, $R^2$, $R^3$ and/or $R^4$ are an alkyl group having 2 to 18 C atoms or a phenylmethyl group.

14. A compound of formula I according to claim 11 in which $R^1$ and $R^3$ are identical and $R^2$ and $R^4$ are identical and as defined above.

15. A compound of formula I according to claim 11 in which $R^1$ and $R^3$ are alkyl having 2-8 C atoms and $R^2$ and $R^4$ are phenylmethyl.

16. A compound of formula I according to claim 11 in which $R^1$, $R^2$, $R^3$ and $R^4$ are each 2-ethylhexyl.

17. A compound of formula I according to claim 11 in which X and $X^1$ are identical and are H, alkyl having 1 to 10 C atoms, phenyl or phenyl substituted by –OH.

18. A compound of formula I according to claim 11 in which X and $X^1$ are H.

19. The compounds of formula I according to claim 11 :
N,S-bis[bis(2-ethylhexyl)aminomethyl]-2,5-dimercapto-1,3,4-thiadiazole and N,S-bis(diallylaminomethyl)-2,5-dimercapto-1,3,4-thiadiazole.

20. Use of a compound of formula I according to claim 11 as a lubricant additive or an additive for hydraulic fluids.

**Revendications**

1. Composition pour lubrifiants ou liquides hydrauliques, composition qui contient au moins un lubrifiant ou un liquide hydraulique et au moins un dimercapto-2,5 thiadiazole-1,3,4 répondant à la formule générale I :

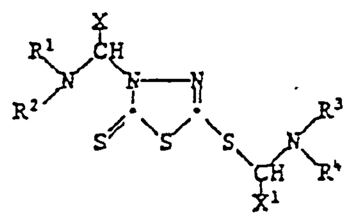

(I)

dans laquelle $R^1$, $R^2$, $R^3$ et $R^4$ représentent chacun, indépendamment les uns des autres, un alkyle contenant de 1 à 24 atomes de carbone, un alcényle contenant de 3 à 12 atomes de carbone, un phénylalkyle dont l'alkyle contient de 1 à 4 atomes de carbone et dont le phényle ne porte pas de substituant ou porte un alkyle en $C_1$-$C_4$, ou

$R^1$ et $R^2$, et/ou $R^3$ et $R^4$, forment ensemble, et avec l'atome d'azote qui les unit un radical de morpholine, de pipéridine, de pyrrolidine, de pipérazine, de méthylpipérazine ou de perhydroazépine, non substitué ou porteur d'un alkyle en $C_1$-$C_4$, et

X et $X^1$ représentent chacun, indépendamment l'un de l'autre, l'hydrogène, un alkyle en $C_1$-$C_{23}$, un phényle, un phénylalkyle à alkyle en $C_1$-$C_4$, ou un phényle porteur d'un alkyle en $C_1$-$C_{12}$, d'un alcoxy en $C_1$-$C_{12}$, d'un hydroxy, d'un di-($C_1$-$C_4$-alkyl)-amino, d'un halogène et/ou d'un nitro.

2. Composition selon la revendication 1 dans laquelle $R^1$, $R^2$, $R^3$ et $R^4$ représentent chacun un alkyle en $C_2$-$C_{18}$ ou un phényl-($C_1$-$C_4$)alkyle, ou $R^1$ et $R^2$, et/ou $R^3$ et $R^4$, forment ensemble et avec l'atome d'azote qui les unit un radical de morpholine, de pipéridine ou de pipérazine, et X et $X^1$ représentent chacun l'hydrogène, un alkyle contenant de 1 à 10 atomes de carbone, un phényle ou un phényle porteur d'un –OH.

3. Composition selon la revendication 1 dans laquelle $R^1$, $R^2$, $R^3$ et/ou $R^4$ représentent un alkyle contenant de 2 à 18 atomes de carbone, un allyle ou un phénylméthyle.

4. Composition selon la revendication 1 dans laquelle $R^1$ et $R^3$ sont identiques l'un à l'autre, et $R^2$ et $R^4$ de même, et ont chacun les significations qui leur ont été données.

5. Composition selon la revendication 1 dans laquelle $R^1$ et $R^3$ représentent chacun un alkyle contenant de 2 à 8 atomes de carbone, et $R^2$ et $R^4$ chacun un phénylméthyle.

6. Composition selon la revendication 1 dans laquelle $R^1$, $R^2$, $R^3$ et $R^4$ représentent chacun un radical éthyl-2 hexyle.

7. Composition selon la revendication 1 dans laquelle X et $X^1$ sont identiques l'un à l'autre et représentent chacun l'hydrogène, un alkyle en $C_1$-$C_{10}$, un phényle ou un phényle porteur d'un –OH.

8. Composition selon la revendication 1 dans laquelle X et $X^1$ représentent chacun H.

9. Composition selon la revendication 1 qui contient du N,S-bis[bis-(éthyl-2 hexyl)-amino-méthyl] dimercapto-2,5 thiadiazole-1,3,4 et/ou du N,S-bis-(diallylaminométhyl) dimercapto-2,5 thiadiazole-1,3,4.

10. Composition selon la revendication 1 qui contient de 0, 01 à 10% en poids, avantageusement de 0,05 à 5% en poids ou, mieux encore, de 0,1 à 3% en poids, par rapport au lubrifiant, d'au moins un composé de formule 1.

11. Dimercapto-2,5 thiadiazoles-1,3,4 répondant à la formule I :

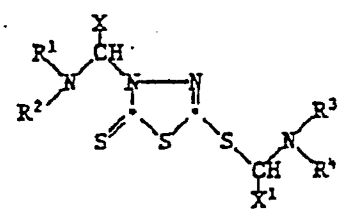

(I)

dans laquelle $R^1$, $R^2$, $R^3$ et $R^4$ représentent chacun, indépendamment les uns des autres, un alkyle contenant de 1 à 24 atomes de carbone, un alcényle contenant de 3 à 12 atomes de carbone, un phénylalkyle dont l'alkyle contient de 1 à 4 atomes de carbone et dont le phényle ne porte pas de substituant ou porte un alkyle

en $C_1$-$C_4$, ou
$R^1$ et $R^2$, et/ou $R^3$ et $R^4$, forment ensemble, et avec l'atome d'azote qui les unit un radical de morpholine, de pipéridine, de pyrrolidine, de pipérazine, de méthylpipérazine ou de perhydroazépine, non substitué ou porteur d'un alkyle en $C_1$-$C_4$, et
X et $X^1$ représentent chacun, indépendamment l'un de l'autre, l'hydrogène, un alkyle en $C_1$-$C_{23}$, un phényle, un phénylalkyle à alkyle en $C_1$-$C_4$, ou un phényle porteur d'un alkyle en $C_1$-$C_{12}$, d'un alcoxy en $C_1$-$C_{12}$, d'un hydroxy, d'un di-($C_1$-$C_4$-alkyl)-amino, d'un halogène et/ou d'un nitro,
avec la condition que, dans le cas où X représente l'hydrogène, ni les radicaux $R^1$ et $R^2$ avec l'azote qui les unit, ni les radicaux $R^3$ et $R^4$ avec l'azote qui les unit, ne représentent en même temps un radical morpholino.

12. Composés de formule I selon la revendication 11, dans lesquels $R^1$, $R^2$, $R^3$ et $R^4$ représentent chacun un alkyle en $C_2$-$C_{18}$ ou un Phénylalkyle à alkyle en $C_1$-$C_4$, ou $R^1$ et $R^2$, et/ou $R^3$ et $R^4$, forment ensemble et avec l'atome d'azote qui les unit un radical de morpholine, de pipéridine ou de pipérazine non substitué ou porteur d'un alkyle en $C_1$-$C_4$, X et $X^1$ représentent chacun l'hydrogène, un alkyle contenant de 1 à 10 atomes de carbone, un phényle ou un phényle porteur d'un OH.

13. Composés de formule I selon la revendication 11, dans lesquels $R^1$, $R^2$, $R^3$ et/ou $R^4$ représentent un alkyle contenant de 2 à 18 atomes de carbone ou un phénylméthyle.

14. Composés de formule I selon la revendication 11, dans lesquels $R^1$ et $R^3$ sont identiques l'un à l'autre, de même que $R^2$ et $R^4$, et ont chacun les significations indiquées ci-dessus

15. Composés de formule I selon la revendication 11, dans lesquels $R^1$ et $R^3$ représentent chacun un alkyle contenant de 2 à 8 atomes de carbone, et $R^2$ et $R^4$ chacun un phénylméthyle.

16. Composés de formule I selon la revendication 11, dans lesquels $R^1$, $R^2$, $R^3$ et $R^4$ représentent chacun un radical éthyl-2 hexyle.

17. Composés de formule I selon la revendication 11, dans lesquels X et $X^1$ sont identiques l'un à l'autre et représentent chacun l'hydrogène, un alkyle en $C_1$-$C_{10}$, un phényle ou un phényle porteur d'un –OH.

18. Composés de formule I selon la revendication 11, dans lesquels X et $X^1$ représentent chacun l'hydrogène.

19. Composés de formule I selon la revendication 11, en l'espèce :
le N-S-bis-[bis-(éthyl-2 hexyl)-aminométhyl] dimercapto-2,5 thiadiazole-1,3,4 et le N-S-bis-(diallylaminométhyl) dimercapto-2,5 thiadiazole1,3,4.

20. Application de composés de formule I selon la revendication 11, comme additifs pour lubrifiants ou additifs pour liquides hydrauliques.